# EUROPEAN PATENT SPECIFICATION

(11) **EP 1 292 319 B1**
(45) Date of publication and mention of the grant of the patent: **16.11.2005**
(21) Application number: 01934245.0
(22) Date of filing: 29.05.2001
(51) Int. Cl.: A61K 35/78, A61P 35/00

(54) **EXTRACTS FROM SPERMATOPHYTE PLANTS WITH ANTITUMOR ACTIVITY**
SPERMATOPHYTE PFLANZENEXTRAKTE MIT ANTITUMORALER WIRKUNG
PROCEDE POUR L'EXTRACTION DE PRODUITS PHARMACEUTIQUEMENT ACTIFS A PARTIR DE PLANTES SPERMATOPHYTES, PRODUITS AINSI OBTENUS ET LEUR UTILISATION EN MEDECINE, NOTAMMENT COMME SUBSTANCES AVEC UNE ACTIVITE ANTITUMORALE

(30) Priority: 30.05.2000 IT RM000293
(43) Date of publication of application: 19.03.2003
(73) Proprietor: CONSIGLIO NAZIONALE DELLE RICERCHE, I-00100 Roma (IT); Istituto Agrario di S. Michele All'Adige, 38010 S. Michele All'Adige (IT)
(72) Inventor: RAVAGNAN, Giampietro, I-00164 Roma (IT); FALCHETTI, Roberto, I-00124 Roma (IT); LANZILLI, Giulia, I-00141 Roma (IT); FUGGETTA, Maria, Pia, I-00179 Roma (IT); TRICARICO, Maria, I-00143 Roma (IT); MATTIVI, Fulvio, 38042 Baselga di Pine' (IT)
(74) Representative: Gervasi, Gemma
(86) International application number: PCT/IB2001/000981
(87) International publication number: WO 2001/091763

(56) References cited:
- WO-A-00/37021
- WO-A-01/03713
- WO-A-01/30336
- WO-A-99/58119
- MATTIVI FULVIO ET AL: "Isolation, characterization, and evolution in red wine vinification of resveratrol monomers." JOURNAL OF AGRICULTURAL AND FOOD CHEMISTRY, vol. 43, no. 7, 1995, pages 1820-1823, XP002196379 ISSN: 0021-8561
- KORHAMMER SIEGFRIED ET AL: "An oligostilbene from Vitis roots." PHYTOCHEMISTRY (OXFORD), vol. 38, no. 6, 1995, pages 1501-1504, XP000946048 ISSN: 0031-9422
- VASTANO BRET C ET AL: "Isolation and identification of stilbenes in two varieties of Polygonum cuspidatum." JOURNAL OF AGRICULTURAL AND FOOD CHEMISTRY, vol. 48, no. 2, February 2000 (2000-02), pages 253-256, XP002182532 ISSN: 0021-8561
- CHEN YONG ET AL: "2,2-diphenyl-1-picrylhydrazyl radical-scavenging active components from Polygonum multiflorum Thunb." JOURNAL OF AGRICULTURAL AND FOOD CHEMISTRY, vol. 47, no. 6, June 1999 (1999-06), pages 2226-2228, XP002196351 ISSN: 0021-8561 cited in the application
- TRELA B C ET AL: "RESVERATROL: ISOMERIC MOLAR ABSORPTIVITIES AND STABILITY" JOURNAL OF AGRICULTURAL AND FOOD CHEMISTRY, AMERICAN CHEMICAL SOCIETY. WASHINGTON, US, vol. 44, no. 5, 1 May 1996 (1996-05-01), pages 1253-1257, XP000583846 ISSN: 0021-8561
- OHYAMA MASAYOSHI ET AL: "Antitumor agents 200. Cytotoxicity of naturally occurring resveratrol oligomers and their acetate derivatives." BIOORGANIC & MEDICINAL CHEMISTRY LETTERS, vol. 9, no. 20, 18 October 1999 (1999-10-18), pages 3057-3060, XP002196352 ISSN: 0960-894X
- LYNOTT J ET AL: "Antimetastatic and antiproliferative action of resveratrol on melanoma." JOURNAL OF INVESTIGATIVE DERMATOLOGY, vol. 112, no. 4, April 1999 (1999-04), page 570 XP001068561 60th Annual Meeting of the Society for Investigative Dermatology;Chicago, Illinois, USA; May 5-9, 1999 ISSN: 0022-202X
- MCFARLAND M ET AL: "Regulation of growth and apoptosis in melanoma cells by resveratrol." FASEB JOURNAL, vol. 14, no. 4, 15 March 2000 (2000-03-15), page A240 XP001069131 Annual Meeting of Professional Research Scientists: Experimental Biology 2000;San Diego, California, USA; April 15-18, 2000 ISSN: 0892-6638
- DAVIS T ET AL: "Investigating chemotherapeutic potential of resveratrol against melanoma." JOURNAL OF INVESTIGATIVE DERMATOLOGY, vol. 114, no. 4, April 2000 (2000-04), page 847 XP001068560 61st Annual Meeting of the Society for Investigative Dermatology.;Chicago, Illinois, USA; May 10-14, 2000 ISSN: 0022-202X
- MEISHIANG ET AL: "Cancer Chemopreventive Activity of Resveratrol, a natural product Derived from grapes" SCIENCE, AMERICAN ASSOCIATION FOR THE ADVANCEMENT OF SCIENCE,, US, vol. 275, 10 January 1997 (1997-01-10), pages 218-220, XP002124508 ISSN: 0036-8075 cited in the application
- XIONG Y P ET AL: "Molecular targets of Resveratrol on human colon cacncer cell lines." PROCEEDINGS OF THE AMERICAN ASSOCIATION FOR CANCER RESEARCH ANNUAL, vol. 40, March 1999 (1999-03), page 55 XP001063169 90th Annual Meeting of the American Association for Cancer Research;Philadelphia, Pennsylvania, USA; April 10-14, 1999, March, 1999 ISSN: 0197-016X
- TESSITORE L ET AL: "Resveratrol inhibits colorectal carcinogenesis induced by azoxymethane." PROCEEDINGS OF THE AMERICAN ASSOCIATION FOR CANCER RESEARCH ANNUAL, vol. 40, March 1999 (1999-03), page 363 XP001063168 90th Annual Meeting of the American Association for Cancer Research;Philadelphia, Pennsylvania, USA; April 10-14, 1999, March, 1999 ISSN: 0197-016X
- DELMAS DOMINIQUE ET AL: "Resveratrol, an efficient inhibitor of human colorectal cancer cell proliferation." BIOLOGY OF THE CELL (PARIS), vol. 92, no. 2, April 2000 (2000-04), page 163 XP001069130 Congress of the French Society of Cell Biology;Paris, France; May 24-26, 2000 ISSN: 0248-4900

## Description

### Field of the invention

The present invention relates to a method for the extraction of pharmaceutically active products from spermatophyte plants, to the products thus obtained and to their use in the medical field, in particular as substances with anti-tumoral activity.

In particular, the invention relates to the use in the pharmaceutical field with anti-tumoral activity of cis-resveratrol (indicated below also as C-Res) and of hydroxylated stilbenes, oligostilbenes and stilbenoids both in their free and glucosidated forms.

### State of the art

Hydroxylated stilbenes, both monomer and oligomer, represent a class of chemical compounds which are present in a limited number of spermatophyte plants and in particular in vine, where they are essential components of the root, stem, leaves and mainly of fruits (Vrhovsek U, Mattivi F, 1998, *Proceedings of the 29*^{*th*} *J. Plecnik, Cardiovascular Diseases,* 449-463; Mattivi F et al., 1995, *J Agric Food Chem,* 42, 1820-1823). As their role in plant physiology seems to be mainly the inhibition of the progression of infections caused by fungi, this group of substances has been included among phytoalexines, a class of antibiotics of vegetal origin (Hain R et al, 1990, *Plant Mol Biol,* 15, 325-335).

The results of a series of studies indicate that one of the compounds belonging to the class of stilbenes, trans-resveratrol, can perform pharmacological activities in humans. Indeed, these researches have shown that trans-resveratrol has antioxidant properties (Fauconneau B et al, 1997, *Life Sci,* 61, 2103-2110), and can inhibit platelet aggregation (Bertelli A et al, 1996, *Drug Exp Clin Res,* 22, 61-63) and cyclooxygenase activity (Jang M et al, 1997, *Science,* 275, 218-220). Moreover, it has been proved that said compound can inhibit *in vitro* the growth of cells belonging to line MCF-7 derived from mammary adenocarcinoma (Mgbonyebi O P et al, 1998, *Int J Oncology,* 12, 865-869), and in cells belonging to line HL60 (human promyelocytic acute leukemia) it can induce the stop of the cellular cycle in the transition from stage S to stage G2 (Della Ragione F et al, *1998, Biochem Biophys Res Com,* 250, 53-58), and, at high doses, it can induce apoptosis and regulate the expression of CD95L (Clement MV et al, 1998, *Blood*, 92, 996-1002). Eventually, *in vivo* trans-resveratrol has proved to be able to inhibit tumor genesis in a murine model of skin cancer induced by carcinogenic substances (Jang M et al, 1997, *Science,* 275, 218-220).
None of these researches mentioned the pharmacological activity and the corresponding mechanisms of said activity for cis-resveratrol, for glucosides of cis- and trans-resveratrol and for oligostilbenes and stilbenoids, which the authors consider useful as therapeutic remedies in the prevention and treatment of tumors.

WO 01 30336 mentions cis- and trans- resveratrol and corresponding glucosides as active principles for pharmaceutical compositions , in particular for treating tumors.

Ohyama Masayoshi et al. *"Bioorganic & Medicinal Letters".* vol. 9, no. 20, 10.10.1999, pages 3057-3060 discloses the use of naturally occurring resveratrol oligomers and corresponding acetate derivatives as antitumor agents. In particular ε-viniferine, hopeaphenol and Ampelopepsin A are mentioned.

Lynott J. et al. *"Journal of investigative Dermatology"* vol. 112, no. 4 (April 1999) page 570; McFarland M. et al. "*Faseb Journal"* vol. 14, no. 4 (15 March 2000) page A240; Davis T. et al. *"Journal of investigative Dermatology"* vol. 114 no. 4 (April 2000) page 847; Jang et. al. *"Science, American Association for the Advancement of Science*, *US"* vol. 275, (10 January 1997) pages 218-220; Xiong Y. Et al. *"Proceedings of the American Association for Cancer Research Annual"* vol. 40, (March 1999) page 55; Tessitore L. et al. *"Proceedings of the American Association for Cancer Research Annual"* vol. 40, (March 1999) page 363; Delmas D. et al. *"Biology of the Cell (Paris)"* vol. 92, no. 2 (April 2000) page 163; the above documents generically mention resveratrol or trans-resveratrol as an antitumor agent.

As is known, at present pharmacological therapy mainly uses drugs performing their cytotoxic activity through various biochemical mechanisms and actions sites directly onto the tumor cell, causing the stop of its replication and its death, and immunotherapeutical drugs acting indirectly, causing for instance the increase of patients' immune responses towards neoplastic cells. Recent researches suggest that substances which can induce apoptosis of neoplastic cells can also play an important role in combined therapy of tumors. Apoptosis, or programmed cell death, is a natural process of genetically regulated "cell suicide", used by multicellular organisms to eliminate unnecessary or old cells, or cells which have been damaged by pathogenic stimuli. Cell death by apoptosis occurs through a series of successive biochemical events such as morphologic (such as for instance blebbing of plasmatic membrane, chromatin condensation and DNA fragmentation) and functional changes of the cell. Experimental evidence has shown that the absence of responses to apoptotic stimuli can be the primary event of tumor genesis and that a series of faults in the apoptotic mechanism found in tumor cells can give said cells resistance both to chemotherapy and to radiotherapy. However, the strategies used at present to identify the factors which can perform one or more of the aforesaid activities have not given final results and their research is one of the most highly developing field of study in the fight against neoplastic illnesses.

### Summary of the invention

An object of the invention is the use of the compounds C-Res, glucosidated C-Res, viniferine, H-gnetine, r-2-viniferine, r-viniferine, hopeaphenol, Ampelopepsin A and glucosidated T-Res, as drugs, in particular as drugs with anti-tumoral activity, more in particular with apoptotic activity.

Other objects will be evident from the description of the invention.

### Brief description of the figures

Fig. 1 shows the effect of C-Res on the replication of M 14 cells.
Fig. 2 shows the effect of C-Res on the apoptosis of M 14 cells.
Fig. 3 shows the effect of C-Res on the mortality of M 14 cells.
Fig. 4A shows the effect of C-Res on the cellular cyde of M 14 cells after 24 hours.
Fig. 4B shows the effect of C-Res on the cellular cycle of M 14 cells after 48 hours.
Fig. 4C shows the effect of C-Res on the cellular cycle of M 14 cells after 72 hours.
Fig. 5 shows the effect of C-Res on the replication of PAR-MEL cells.
Fig. 6 shows the effect of C-Res on the apoptosis of PAR-MEL cells.
Fig. 7 shows the effect of C-Res on the mortality of PAR-MEL cells.
Fig. 8A shows the effect of C-Res on the cellular cycle of PAR-MEL cells after 24 hours.
Fig. 8B shows the effect of C-Res on the cellular cycle of PAR-MEL cells after 48 hours.
Fig. 8C shows the effect of C-Res on the cellular cycle of PAR-MEL celts after 72 hours.

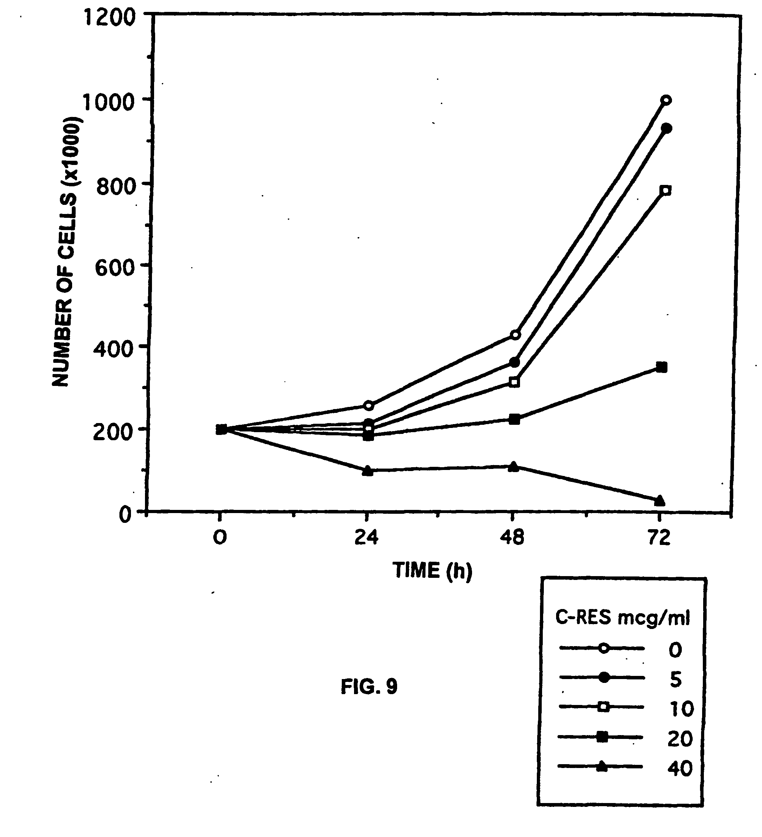

Fig. 9 shows the effect of C-Res on the replication of HT-29 cells.
Fig.10 shows the effect of C-Res on the apoptosis of HT-29 cells.
Fig. 11 shows the effect of C-Res on the mortality of HT-29 cells.
Fig. 12A shows the effect of C-Res on the cellular cycle of HT-29 cells after 24 hours.
Fig. 12B shows the effect of C-Res on the cellular cycle of HT-29 cells after 48 hours.
Fig. 12C shows the effect of C-Res on the cellular cycle of HT-29 cells after 72 hours.
Fig. 13 shows the comparison between the anti-tumoral activity of cis-resveratrol (C-Res) and of trans-resveratrol (T-Res).
Fig. 14 shows the reverse-phase HPLC chromatographic plot of the complex mixture of stilbenes, oligostilbenes and stilbenoids extracted from Vitis roots: trans-stilbenes and trans-oligostilbenes are monitored at 320 nm, cis-stilbenes and stilbenoids at 282 nm.

### Detailed description of the invention:

The compounds used in the present invention can be roughly divided into two families: the first is the one of stilbenes and oligostilbenes, the second is the one of stilbenoids. The first one comprises molecules characterized by the presence of one or more stilbene groups [(C₆H₅)-CH=CH-(C₆H₅)], variously hydroxylated and/or glucosidated. As a mere non-limiting example, the following compounds belong to said first family: 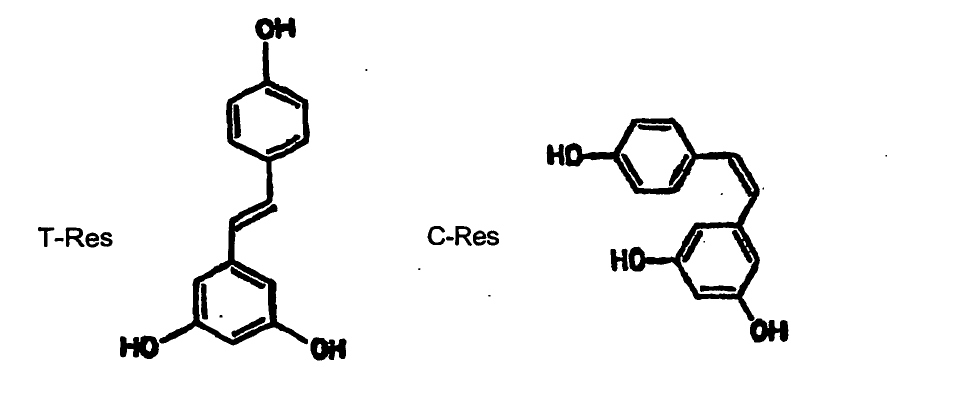 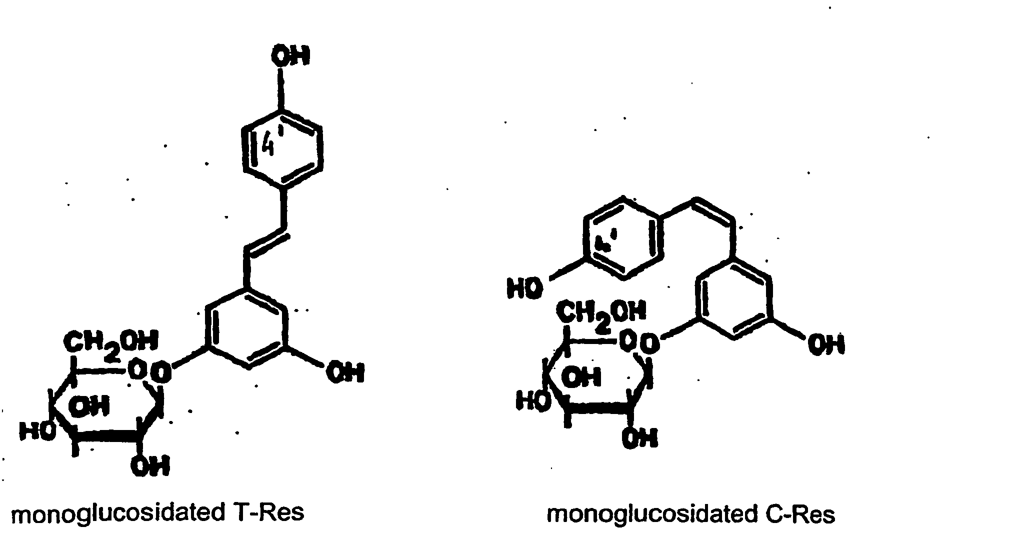 (alternatively to position 3, the glucosidic bond can also be present in position 4' and result in different T-Res and C-Res, differently glucosidated, or T-Res and C-Res having more than one glucosidic group).

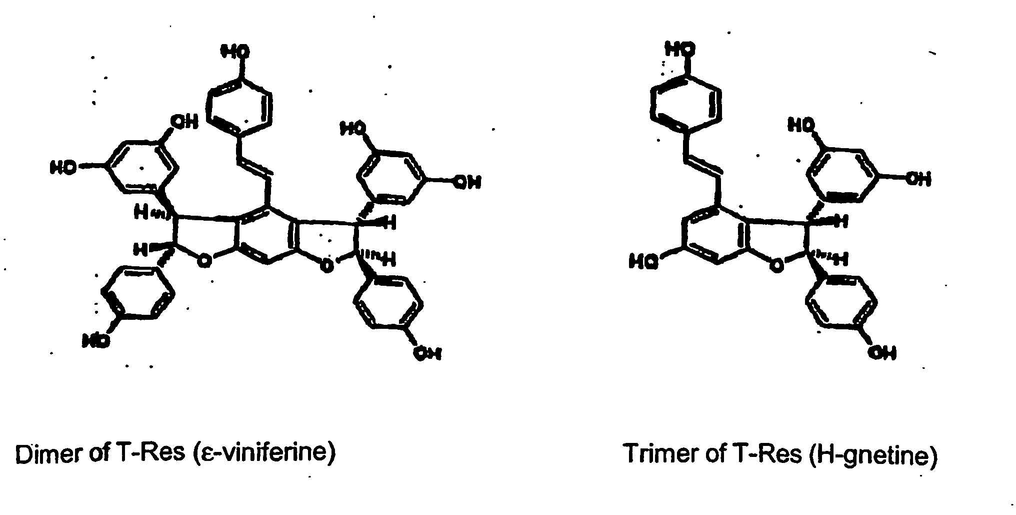

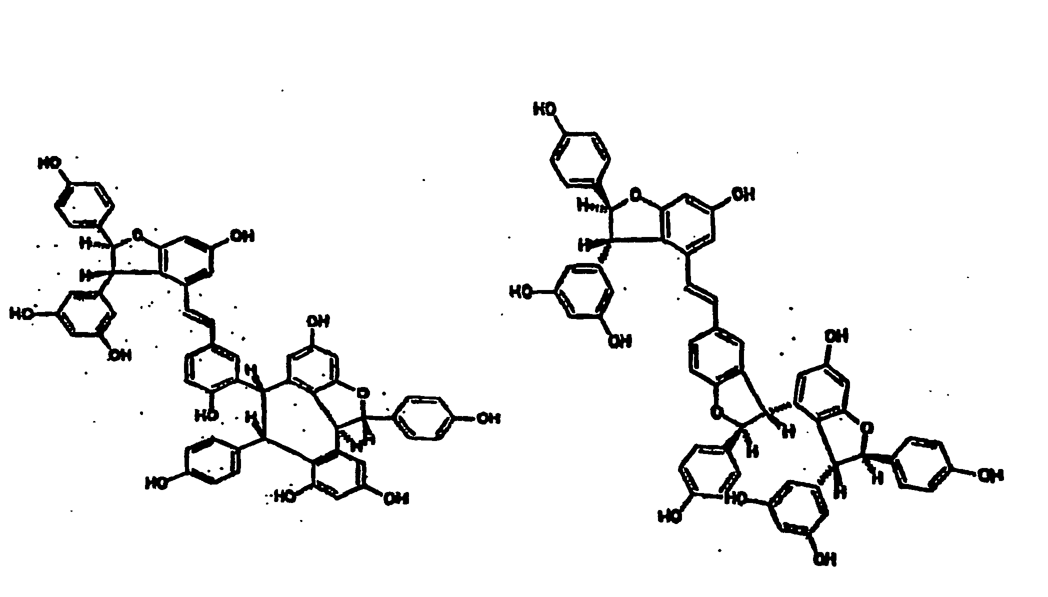

### Tetramers of T-Res (r-2-viniferine and r-viniferine)

The second family, the one of stilbenoids, comprises compounds which, like the previous ones, can be classified as stilbene oligomers, but in which the original stilbene structure is not recognizable, since all stilbene double bonds have been modified by natural enzymatic biosynthetical processes within the vegetal products they are extracted from. As a mere non-limiting example the following compounds can be mentioned:

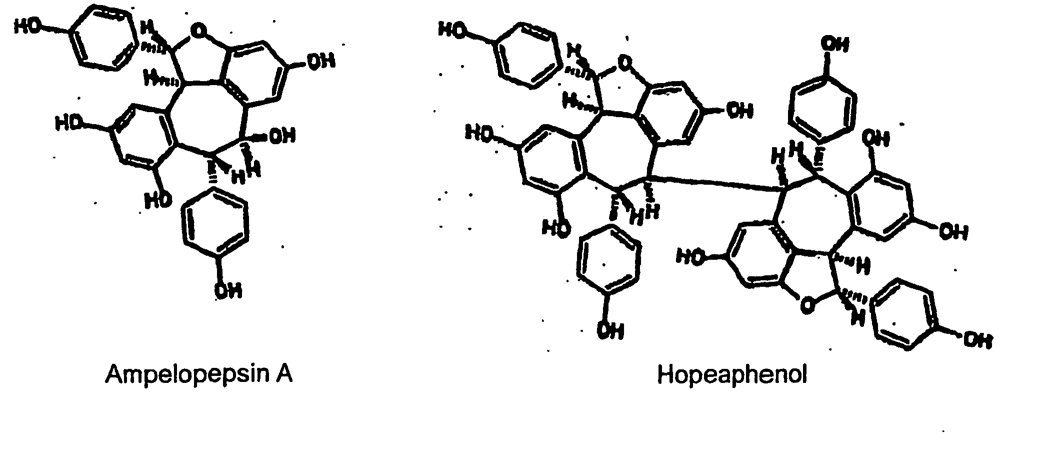

Ampelopepsin A is considered as an oxidative dimer of T-Res. Hopeaphenol is the corresponding tetramer, i.e. a dimer of dimers which are bonded through two C8 positions.

The compounds belonging to the two classes can be obtained by means of extraction and purification treatments from the natural products containing them, for instance fruits (ex. grapes), aerial parts (trunk, shoots, leaves) or subterranean parts of spermatophyte plants, in particular those belonging to the family of Vitaceae or Polygonaceae. Subterranean parts of such plants are particularly preferred.

Extraction and purification process follow the methods indicated below. It should be kept in mind that the mixtures obtained with the process according to the present invention are complex mixtures within which only the main components have been identified (stilbenes, oligostilbenes and stilbenoids indicated above): however, these products have to be regarded only as representative of other similar components, which are present in smaller quantities and which have not been isolated and characterized though they have the same nature and features of applications; these products are also part of the present invention, though not isolated and characterized, since they are present in natural extracts.

The present invention relates to the anti-tumoral activity carried out by these compounds, both alone and in mixture, through their ability to inhibit cell replication, to induce apoptosis and mortality and to induce at various levels the block of the cellular cycle in cells belonging to different tumor lines, and in particular in cells of malignant melanoma resisting to chemotherapeutic treatments.

The extraction process according to the present invention is applied to spermatophyte plants. The starting material can be fruits, aerial parts or subterranean parts of the plants. The starting material can be fresh or frosted or lyophilized and pulverized. The most significant matrixes are considered to be the roots of Polygonum cuspidatum and of Polygonum multiflorum and the bark of lignified root of the genus Vitis.

It is preferable to work with materials which are as dry as possible, and so it can be advantageous to subject said material to be extracted to a pre-treatment including the substantial cold elimination of water, for instance by lyophilization.

The extraction is carried out in a neutral environment with an aliphatic alcohol, preferably methanol or ethanol and their mixtures, preferably using solvent amounts between 10 to 20 times by volume the weight of the matrix to be extracted. The material to be extracted and the solvent are mixed, the whole is agitated and extracted in oxygen-free atmosphere, for instance saturated with nitrogen, and light-shielded, at room temperature, with a duration of the extraction varying according to the matrix, generally around 2-12 hours for matrixes as such, and around 5-120 minutes for matrixes which have been previously lyophilized and pulverized by previously grinding the lyophilized product. The final extract is centrifuged and the supernatant liquor is recovered, concentrated under vacuum at low temperatures (lower than 45°C) and taken up with ethyl acetate or another similar solvent such as for instance methyl acetate and/or tetrahydrofuran (raw extract in solvent) or with water (aqueous raw extract) (in particular for Polygonum roots, so as to obtain a higher purity degree).

The raw extract can be treated following one of two methods, depending whether a mixture substantially containing all the products according to the present invention or only trans-resveratrol and glucosidated trans-resveratrols is to be obtained.

The first kind of treatment (Treatment A) is preferred in case the extraction is carried out on plants of the genus Vitis, whereas the second kind of treatment (Treatment B) is preferred for the simpler matrix related to plants of the genus Polygonaceae.

Treatment (A) The raw extract in a solvent such as ethyl acetate is washed with water saturated with an inorganic salt (for instance NaCl), the fraction in ethyl acetate is loaded onto a column prepared with a resin of an aromatic polymer (stirene-divinylbenzene copolymers, preferably with particle size between 0.1 and 0.25 mm, are particularly suitable to this purpose).

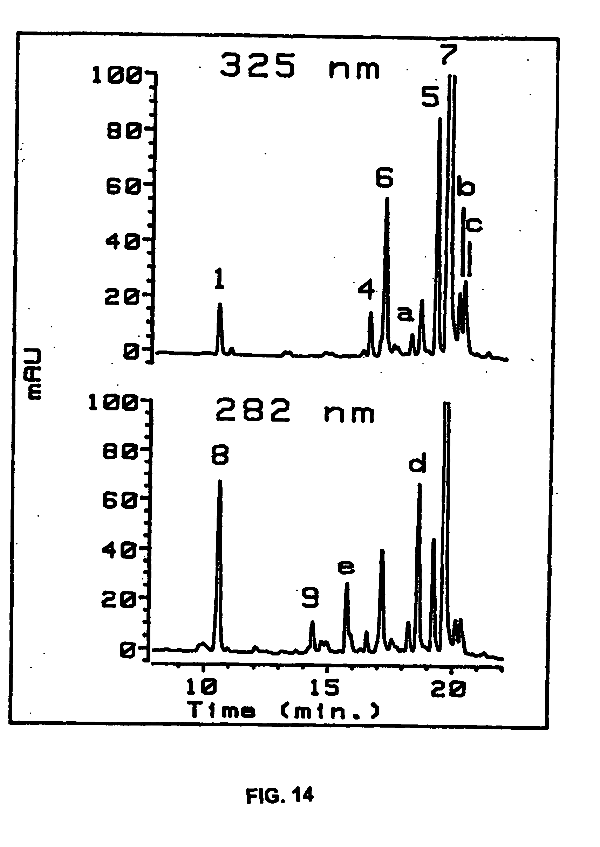

After the loading the whole is eluted with water, preferably in a volume which is about twice that of ethyl acetate, then with pentane-methylene chloride 2:1 (in a volume which is almost as much as that of ethyl acetate). Stilbenes are eluted with ethyl acetate or with suitable mixtures of organic solvents with intermediate polarity, whose eluting strength corresponds to XAD-2, an abbreviation which is known to the person skilled in the art. XAD-2 is a particular kind of stirene-divinylbenzene copolymer resin with respect to which the factors concerning relative eluting strength are tabulated in the scientific literature (see e.g. Robinson J.L. et al., *J*. *Chromatogr.,* 1980, 185, 145), such as for instance tetrahydrofuran and methylene chloride. The volume of ethyl acetate is chosen so as to ensure the quantitative recovery of the whole class (as in Fig. 14) and varies according to the shape and free volume of the column. The product of this selective elution consists of a purified fraction containing the whole class of viniferines, containing both oligostilbenes and stilbenoids.

The basic constituents ensuring a pharmacological interest to this fraction are the oligomers of resveratrol, with particular attention to dimers, trimers and tetramers, both those still containing a stilbene double bond in trans or cis form (oligostilbenes) and those which have lost their stilbene structure during natural polymerization (stilbenoids). The same fraction also contains the monomer of glucosidated trans-resveratrol.

If the following pure compounds are to be obtained: epsilon-viniferine, alfa-viniferine, gnetina-H (which can also be isolated from wood of Welwitschia mirabilis),r-viniferine, Ampelopepsin A (which can also be isolated from roots of Ampelopsis brevipedunculata) and hopeaphenol, a high-performance liquid chromatography on reverse-phase columns has to be carried out. Phases based on silica functionalized with C18 o C8 (terms known to the person skilled in the art), or stirene polymers can be used, the latter only with low pressure. A practical example (ideal to separate epsilon-viniferine, H-gnetine and r-viniferine) is the use of a packed column with fixed phase RP-18, for instance LiChrospher 100 or similar, particle size 10 microns, eluting with a linear gradient of water and acetonitrile, the latter 30 to 50%. The setup of the separation conditions for this kind of mixtures is known to the person skilled in the art.

A quantitative analysis of the mixture of stilbenes, oligostilbenes and stilbenoids obtained with the extraction according to the invention can be generally obtained by reverse-phase high-performance liquid chromatography (or with other separation techniques in liquid phase such as electrophoresis, thin layer chromatography (TLC) with UV detection, MS (Mass Spectrometry) or fluorescence detector. The first two techniques are preferred since they allow a reliable identification. An example of optimized conditions can be found in example 8.

### Treatment B

Treatment B consists of the following alternatives:
- if both lipophilic and hydrophilic compounds have to be eliminated from the final extract, the aqueous raw extract is washed with methylene chloride or other solvent with similar polarity such as for instance chloroform, in order to remove lipophilic products though not stilbenes, and is then re-extracted in ethyl acetate (for instance five times with extract/extractant volume of 1/1, to be reduced in case of higher volumes of extractant or of modification of the ionic strength of the extract), to recover stilbenes, whereas hydrophilic compounds remain in the water portion which is discarded.
- if only glucosidated derivatives have to be obtained from the final extract, these can be selectively cold-precipitated from the raw extract in a solvent such as ethyl acetate with a non polar solvent (hexane would be ideal, in a 3/1 ratio with respect to ethyl acetate), and recovered by filtration. Alternatively, the whole fraction can be used in order to recover trans-resveratrol as well.
- if a quite accurate separation of the mixture has to be obtained, it is possible to start from the raw extract in a solvent such as ethyl acetate, which should be perfectly anhydrous. Said raw extract, preferably reduced to its minimum volume, is loaded to the head of a preparative silica column for chromatography (for instance Kieselgel 0.05-0.20 mm), the latter being packed in a non polar solvent (for instance hexane). A suitable volume of chloroform is left above the column. The treatment provides for two subsequent washing and elution sequences with mixtures having increasing eluting strengths, chloroform-methanol, I (20:1); II (10:1); III (5:1); IV (2.5:1) respectively. More generally, with suitable binary or ternary mixtures of solvents with moderate polarity (for instance chlorinated solvents, diethyl ether, tetrahydrofuran) and strong solvents (for instance aliphatic alcohols, acetonitrile) it is possible to obtain similar separations using mixtures having a position in the elution series which is wholly similar to the ones indicated above, according to what is known to the person skilled in the art. This separation enables an integral recovery of stilbene active agents, separated from all major interfering substances, thus obtaining two preparations with very high purity: fraction IV contains two different monoglucosidated derivatives of trans-resveratrol, gathered into the same fraction, and fraction II contains free trans-resveratrol. The conditions described here refer in particular to the extract of roots of Polygonum cuspidatum, and can be adapted with a similar procedure to Polygonum multiflorum, which contains - as is known - also other kinds of glucosidated stilbenes (2,3,5,4'-tetrahydroxystilbene-2-glucoside, Yong et al., 2,2-*diphenyl-1-picriylhydrazyl radical-scavenging active components from Polygonum multiflorum Thunb., J. Agr. Food Chem.,* 1999, 47, 226-2228). If it is necessary to go as far as the molecular level, purification techniques for preparative chromatography can be used, in the conditions described in Mattivi et al., *Isolation*, *characterization and evolution in red wine vinification of resveratrol monomers, J. Agr. Food Chem.,* 1995, 43, 7, 1820-1823.

A quantitative analysis of resveratrols can be carried out according to example 8. The techniques for quantitative analysis described are particularly indicated for instance to evaluate the presence of the desired active agent in culture mediums, and therefore to support in vitro experimental models.

### Trans-cis isomerization

An indication of isomerization techniques according to the invention can be found in example 7.

### Therapeutic activity

In the therapy it is possible to use extracts as such, or the individual compounds both in natural form (as isolated from the matrixes of vegetal origin) and as obtained from chemical synthesis. The active agent can be administered in form of pharmaceutically acceptable salt, ester, amide, prodrug or similar or their combinations. Salts, esters, amides, prodrugs or similar of the active agents can be prepared by following the standard procedures of organic synthetic chemistry.

On the basis of the administration form which is going to be used, the pharmaceutical formulation can be in form of tablets, suppositories, pills, capsules, powders, liquids, suspensions, creams, ointments, lotions or similar. The compositions will therefore include an effective amount of the agent in combination with a pharmaceutically acceptable excipient and can also include other pharmacological agents, adjuvants, diluents, buffers, etc. The compounds can then be administered by oral, parenteral (subcutaneous, intravenous, intramuscular injection), transdermic, rectal, nasal, buccal way, by topical administration or by means of a controlled-release implant.

The amount of the active compound to be administered will depend on the particular pathology (or pathologies) affecting the patient to be treated, on the weight and age of the patient, on the kind of administration chosen and on the attending physician's opinion. In the method according to the invention, anti-tumoral activity, the treatment scheme will provide for the administration of the - drug at doses between 0.0001 and 20 mg/kg/die.

It has been verified that the compounds, both alone and in mixture, according to the present invention, in particular C-Res, are inducers of cell apoptosis; moreover, in a dose range of 5 and 10 mcg/ml C-Res performs a significant cytostatic activity, whereas at a dose of 20 and 40 mcg/ml the compound has a cytotoxic activity.

It has also been found that the compounds, both alone and in mixture, according to the present invention, in particular C-Res, can perform an anti-tumoral activity in general and in particular an activity against malignant melanoma resisting to chemotherapeutic treatments and against colorectal adenocarcinomata. Moreover, the specific anti-tumoral activity of C-Res has proved to be at least 12 times higher than that of T-Res.

The following non-limiting examples will illustrate the present invention.

### Example 1 (Not part of the invention)

### Extraction of resveratrols (trans-resveratrol, cis-resveratrol and their glucosides) from vegetal matrixes (fruits, vine grapes)

The matrix is frozen and then homogenized in presence of sodium metabisulfite and ascorbic acid in an amount of 1% by weight each, the homogenized product undergoes liquid-liquid extraction three times with ethyl acetate (150% by volume with respect to the matrix weight) in absence of light. The extracts are washed once with a 3% water solution of sodium bicarbonate and twice with distilled water, with a volume of 10% of the extract volume for each washing. The washed extract is anhydrified with anhydrous sodium sulfate or by freezing and concentrated until dryness under reduced pressure and at low temperature. The dry extract is taken up with anhydrous ethyl acetate.

### Example 2 (Not part of the invention)

### Extraction of resveratrols (trans-resveratrol, cis-resveratrol and their glucosides) from root of Polygonum cuspidatum

The root of the repotted plant is washed, dried, cut into big pieces, lyophilized and ground. The extraction takes place with methanol (or ethanol) under stirring in absence of light and oxygen. Then the extract is centrifuged and the supernatant liquor is recovered and concentrated under reduced pressure and low temperature and taken up with ethyl acetate.

### Example 3 (Not part of the invention)

### Extraction of viniferine (oligomer stilbenes and stilbenoids) from vegetal matrixes

The extraction of viniferine from aerial parts (trunk, shoots, leaves) or from subterranean parts of plants of the family of Vitaceae or Polygonaceae can be carried out both on fresh or frozen matrix, or on the lyophilized and pulverized part. If the extraction is carried out on the roots, the latter are washed, dried, cut into big pieces, lyophilized and ground. The matrix which is regarded as being the most significant consists of the bark of lignified roots of the genus Vitis.

The extraction is obtained with methanol (or alternatively with ethanol) in a volume which is 10 to 20 times the weight of the matrix to be extracted, in an oxygen-free, e.g. saturated with nitrogen, and light-shielded atmosphere at room temperature, with a duration varying according to the matrix. The final extract is concentrated under reduced pressure and at low temperature and taken up with ethyl acetate.

### Example 4 (Not part of the invention

### Purification of viniferine, preparation of the purified extract containing the whole class of these compounds

The concentrated extract obtained is washed with water saturated with an inorganic salt (for instance NaCl), the fraction in ethyl acetate is loaded onto a column prepared with resin of a stirene-divinylbenzene polymer, with particle size between 0.1 and 0.25 mm, then pre-purified through consecutive washings with methanol, methylene chloride, acetone, methanol, water. A volume of water corresponding to about 10 times the volume of the extract to be loaded is left on the head of the column. After the loading the absorption on the column head is started, followed by washings with water, then with pentane-methylene chloride 2:1. Stilbenes are then eluted with ethyl acetate. The product of this selective elution consists of a purified fraction containing the whole class of viniferine, containing both oligostilbenes and stilbenoids. The other polyphenols, strongly adsorbed, are eluted with methanol and/or methanol acidified with strong mineral acid.

In particular, the basic constituents granting a pharmacological interest to this fraction are the oligomers of resveratrol, with particular attention to dimers, trimers and tetramers, both those still containing a stilbene double bond in trans or cis form (oligostilbenes) and those which have lost their stilbene structure during natural polymerization (stilbenoids). The same fraction also contains the monomer of glucoside trans-resveratrol.

### Example 5 (Not part of the invention)

### Purification of resveratrols, preparation of the purified extract, if possible with separation of free forms from glucosidated forms

The raw extract obtained as described above from roots of Polygonum cuspidatum is treated as follows. If only glucosides have to be purified, these can be selectively cold-precipitated in a non polar solvent (hexane would be ideal). Alternatively, the whole fraction can be used in order to recover trans-resveratrol as well. The extract in ethyl acetate, which should be perfectly anhydrous, is reduced to its minimum volume and loaded into the head of a preparative silica column for chromatography (for instance Kieselgel 0.05-0.20 mm), packed in hexane. A suitable volume of chloroform is left above the column. The treatment provides for two subsequent washing and elution sequences with various chloroform-methanol mixtures, I (20:1); II (10:1); III (5:1); IV (2.5:1) respectively. This separation enables an integral recovery of active agents, separated from all major interfering substances, thus obtaining two preparations with very high purity: one contains two different monoglucosides of trans-resveratrol, gathered into the same fraction, and the other one contains free trans-resveratrol. If it is necessary to go as far as the molecular level, purification techniques for preparative chromatography can be applied, in the conditions described in Mattivi et al., *Isolation, characterization and evolution in red wine vinification of resveratrol monomers, J. Agr. Food Chem.,* 1995, 43, 7, 1820-1823.

### Example 6 (Not part of the invention)

### Preparation of pure compounds of the family of viniferine

If the starting product consists of the roots of Vitis, the following pure compounds can be obtained by means of purification: epsilon-viniferine, alfa-viniferine, gnetina-H (which can also be isolated from wood of *Welwitschia mirabilis),* r-viniferine, Ampelopepsin A (which can also be isolated from roots of *Ampelopsis brevipedunculata)* and hopeaphenol.

The final isolation of these pure compounds can be obtained through high-performance liquid chromatography on reverse-phase columns. Phases based on silica functionalized with C18 o C8, or stirene polymers can be used successfully, the latter only with low pressure. A practical example (ideal to separate epsilon-viniferine, H-gnetine and r-viniferine) is the use of a column packed with LiChrospher 100 RP-18, particle diameter 10 microns, eluting with a linear gradient of water and acetonitrile, the latter 30 to 50%.

### Example 7(Not part of the invention)

### Trans-cis isomerization

This reaction can be used both to obtain products which are not commercially available (example: cis-resveratrol) and to move the balance of mixtures of natural extracts containing both forms, if pharmacologically necessary.

Cis isomers can be prepared by means of photoisomerization starting from the correspondent trans isomers. The best conversion yield can be obtained by feeble irradiation in the near ultraviolet or in the visible light, which can be carried out in vessels of transparent glass. For instance, the isomerization of a solution containing 0.5 mg/ml of commercial T-Res in ethanol, protected from oxygen by means of degassing in an ultrasound bath, insufflated with nitrogen and then sealed and irradiated at 366 nm, allows to obtain conversion yields around 90% with times of 600 minutes. The conversion can be controlled using the techniques of high pressure liquid chromatography (HPLC) described above, by means of direct injection, stopping the reaction when the desired yield has been reached. Obviously, times and modes have to be adapted according to general practice: stronger irradiations can be used only if faster process controls, known to the person skilled in the art, are applied (direct UV, isocratic HPLC), which is easy anyway in case of single compounds. The alcoholic solution, in our case with ethanol, enables an optimal stability of the preparation, which has to be kept in solution since it has great problems of stability in the solid status.

### Example 8(Not part of the invention)

### Methods of quantitative analysis

As far as the quantification of viniferine, in mixture from Example 4 or Example 6, is concerned, a volume between 1 and 6 microlitres of extract in ethyl acetate, anhydrified and filtrated at 0.22 micron, is injected into a HPLC system with reverse-phase column Hypersil ODS C18, 5 micron, 200x2,1 mm. The instrumental conditions provide as eluants A=H₃PO₄ 1 mM in water, B=acetonitrile, linear gradient from 100% A, +2% B/min, 0,6 ml/mm flow, 40°C. The detection is obtained with a photodiode detector at 325 nm for oligostilbenes and at 282 nm for stilbenoids. Quantification with external standard method with reference to calibration straight lines with pure reference compounds.

As far as resveratrols are concerned, they can be obtained with two alternative techniques, HPLC or GC. For both techniques quantification is carried out with the internal standard method. The compound we have found to be optimal is trans-4-hydroxy-stilbene, commercially available.

For GC analyses the techniques described in Mattivi et al. *Isolation*, *characterization, and evolution in red wine vinification of resveratrol monomers, J.*

*Agric. Food Chem.,* 1995, 43, 7, 1820-1823, are used. A derivatizing agent other than those indicated in the related literature has to be used, consisting of trimethylchlorosilane and hexamethyldisilazane in anhydrous pyridine. These specific conditions are determining to overcome the problems of silanization with bis(trimethylsilil)trifluoroacetamide (BSTFA).

For HPLC the reference method with reverse-phase column can be applied, with detection at 310 nm for trans isomers and at 282 for cis isomer (Mattivi F. *Solid* *phase extraction of trans-resveratrol from wines for HPLC analysis. Zeitschrift für Lebensmittel-Untersuchung und Forschung,* 1993, 196, 522-525). The instrumental conditions are the same as those described above for viniferine. The coefficient of molar extinction of both glucosides is almost the same as the one of the corresponding aglycones, so that they can be determined as such after carrying out a correction for molecular weights (Mattivi et al. *Isolation, characterization, and evolution in red wine vinification of resveratrol monomers, J*. *Agric. Food Chem*., 1995, 43, 7, 1820-1823).

If a preparation for aqueous or hydroalcoholic matrixes is necessary, the injection should be preceded by a pre-purification step on reverse-phase cartridges according to Mattivi F. *Solid phase extraction of trans-resveratrol from wines for HPLC analysis. Zeitschrift für Lebensmittel-Untersuchung und Forschung,* 1993, 196, 522-525. In addition to what is contained in the aforesaid document, it has been verified that the method is suitable for all monomer stilbenes, both free and glucosides, and that the eluted fraction of ethyl acetate can be dried under a mild flow of nitrogen, in case it is necessary to carry out the exchange in another solvent and/or to further reduce the limits of quantification.

Said techniques can be used for instance to control the stability of the active agent in culture mediums, and therefore to support in vitro experimental models which are going to be described in the following examples. For instance, for the culture means described in ex. 10 it is possible to find halving times of 38 h for cis-resveratrol, which is still present in an amount of about 25% at the end of the test (72 h), whereas the halving time of 28 h is still high for trans-resveratrol, which is not present as such after 72 h.

### Example 9 (Not part of the invention)

### Methods of qualitative analysis

The main experiments which are necessary for univocal characterization at the molecular level are here recalled according to the technique they use:
Ultraviolet spectroscopy: in a UV spectrophotometer with static cell the double measurement is carried out in absolute ethanol without and with the addition of sodium ethylate (Hillis W.E., Ishikura N. 1968 *J. Chromatogr.,* 32, 323). In a photodiode detector coupled with HPLC, according to Mattivi F., Raniero F. *Relationship between UV spectra and molecular structure of resveratrol oligomers. Polyphenols Communications* 96, 125-126. Given y=A230/A236, the number of stilbene units in the oligomers, i.e. x, is obtained with the equation y=0.657x - 0.065.
Nuclear magnetic resonance: 1 H in NMR spectrometer at 400 MHz or higher, 13C at 100 MHz or higher, in acetone hexadeuterated with TMS as reference. As an alternative, resonances of the methyl group of hexadeuterated acetone can be used as reference (deltaH=2.04 deltaC=29.8). For the specific compounds, according to the requirements, various measuring experiments can be applied, e.g. homonuclear DEPT, 1 H COSY, 1 H COSY long range, double quantum filtered COSY, heteronuclear HECTOR and heteronuclear long range 1H-13C(13C)-GARP decoupling experiments, HMQC, HMBC, NOE difference spectra. Optimal concentrations for measurements in 5 mm probes are 15 mg in 0.5 ml for proton experiments, and 80 mg in 0.5 ml for carbon experiments.
Mass spectrometry, in particular FAB-MS registered in negative modality, in glycerol matrix.
Infrared spectrometry: registered in KBr tablets.
Acetylation of free hydroxyls, in the conditions indicated by Koenig et al. (1987, *Phytochemistry,* 26, 2, 423-427).

### Example 10

### Experimental models for the study of anti-tumoral activity

### Cell lines and treatments

During the experiments a series of cell lines originating from different types of tumors has been used. All cell lines have been grown and kept in a RPMI-1640 medium added with 10% fetal calf serum (FCS) and 1% penicillin-streptomycin. The substances according to the invention have been dissolved in dimethylsulfoxide (DMSO) at an initial concentration of 100mM and added to the cell cultures in the concentrations indicated. DMSO alone has been added to control cultures.

### Determination of cell replication

The cells placed on a microplate in a concentration of 1×10⁵/ml have been incubated with various doses of each substance and for different periods of time. At the end of the treatment cell replication has been evaluated by a cellular count carried out using a Coulter Counter.

### Determination of cell mortality

The cells placed on a microplate in a concentration of 1×10⁵/ml have been incubated with various doses of each substance and for different periods of time. At the end of the treatment cell mortality has been evaluated by a cellular count in trypan blue and/or by flow cytometry using a cytometer FACscan (Becton-Dickinson), determining the vitality of unfixed cells which have been marked with propydium iodide (PI).

### Determination of apoptosis by flow cytofluorometry

The cells have been resuspended in a concentration of 1×10⁶/ml and incubated for different periods of time and with various doses of each substance. At the end of the incubation period the cells have been fixed in acetone/methanol 1:4, treated with 100 KU/ml of RNase and marked with propydium iodide (50 mcg/ml). The cells have then been analyzed by means of flow cytofluorometry using a cytometer FACscan.

### Determination of apoptosis by confocal microscopy

The cells have been resuspended in a concentration of 1×10⁶/ml and incubated for different periods of time and with various doses of each substance. At the end of the incubation period the cells have been fixed in 4% paraformaldehyde, treated with 100 KU/ml of RNase and marked with propydium iodide. The cells have then been analyzed by means of observation with a confocal microscope (LEIKA TCS 4D).

### Analysis of cellular cycle

The cells have been resuspended in a concentration of 1×10⁶/ml and incubated for different periods of time and with various doses of each substance. At the end of the incubation period the cells have been fixed in acetone/methanol 1:4, treated with 100 KU/ml of RNase and marked with propydium iodide (50 mcg/ml). The various stages of the cellular cycle have then been analyzed by means of flow cytometry with a cytometer FACscan using a suitable statistic program.

### Example 11

### Anti-tumoral effects of cis-resveratrol on M14 cells (malignant melanoma)

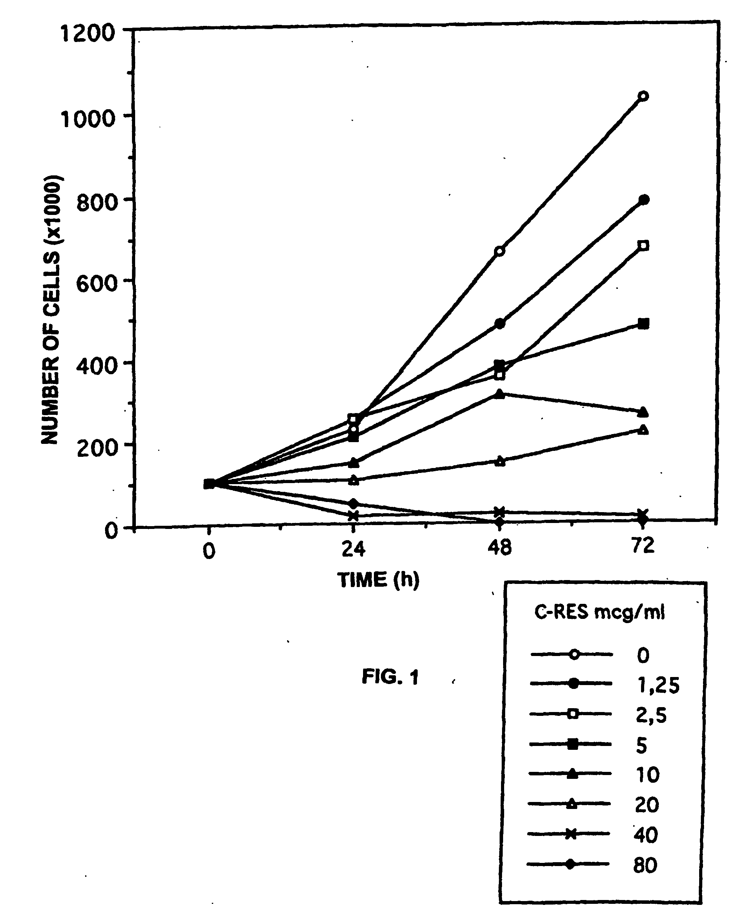

Cells belonging to the M14 tumor line (malignant melanoma) have been incubated for various periods of time with 1.25, 2.5, 5, 10, 20, 40 and 80 mcg/ml of cis-resveratrol (C-Res), or DMSO alone as far as controls are concerned. The results of the experiments show that in all tested doses C-Res can inhibit cell growth. In particular, data show (Fig. 1) that in a dose range of 5-20 mcg/ml C-Res performs a significant cytostatic activity, whereas at a dose of 40 mcg/ml the compound has a cytotoxic activity.

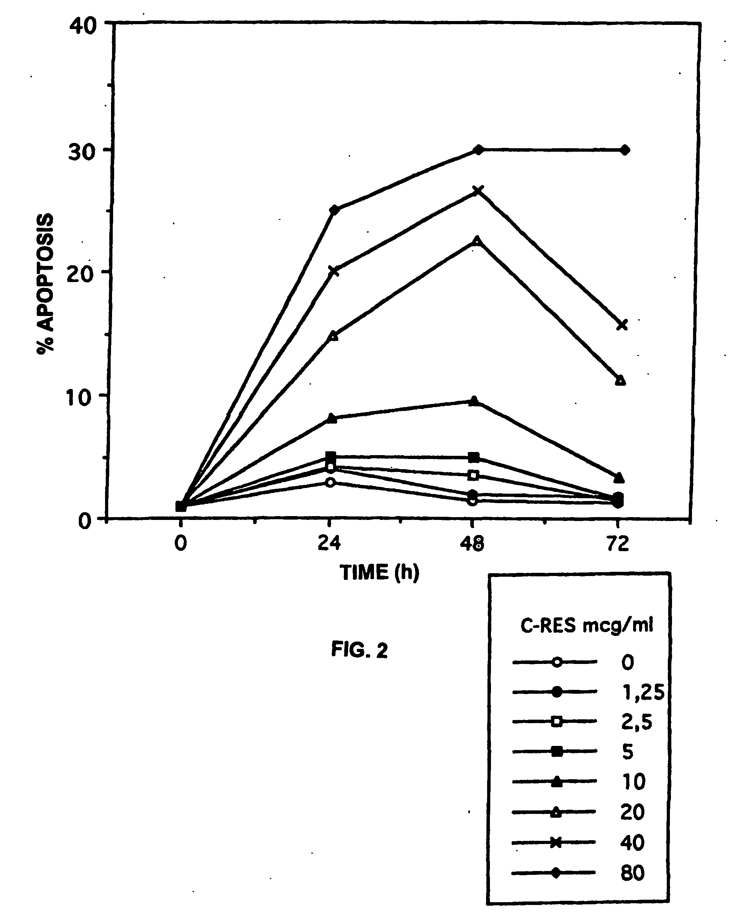

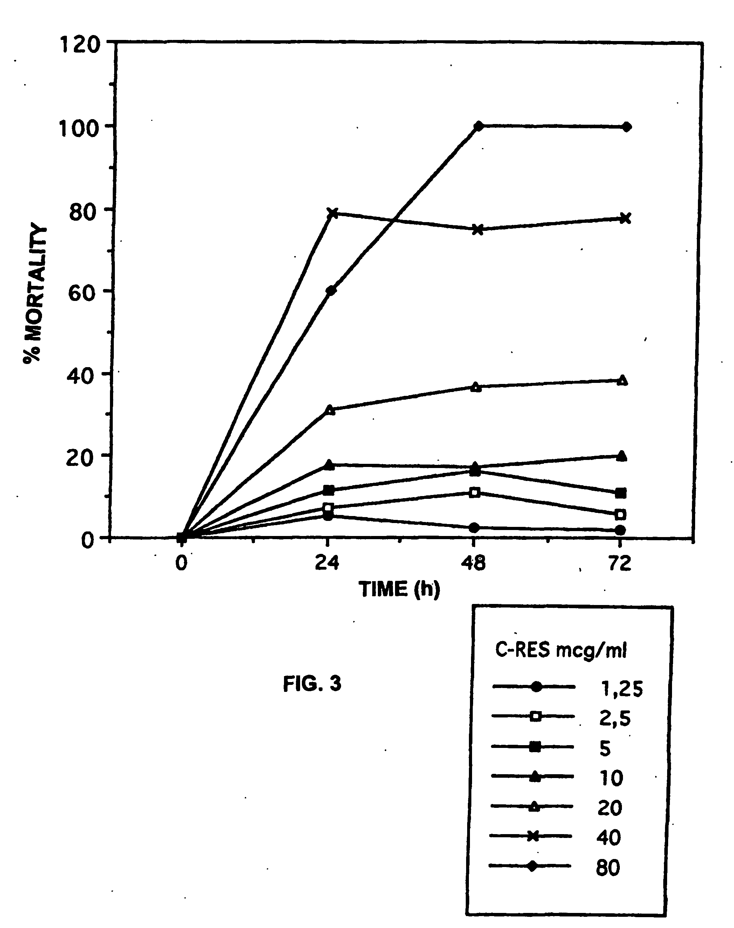

The cytofluorometric analysis of the DNA content of cells marked with PI shows (Fig. 2) that C-Res induces an increase in the number of apoptotic cells depending on the dose and drug exposition time. Said effect is indeed particularly significant starting from a dose of 20 mcg/ml and the peak of activity can be observed after 48h. An activity having a similar course is performed by C-Res on cell mortality (Fig. 3), even though for said parameter the percentages of cells reacting to the action of the drug are extremely higher.

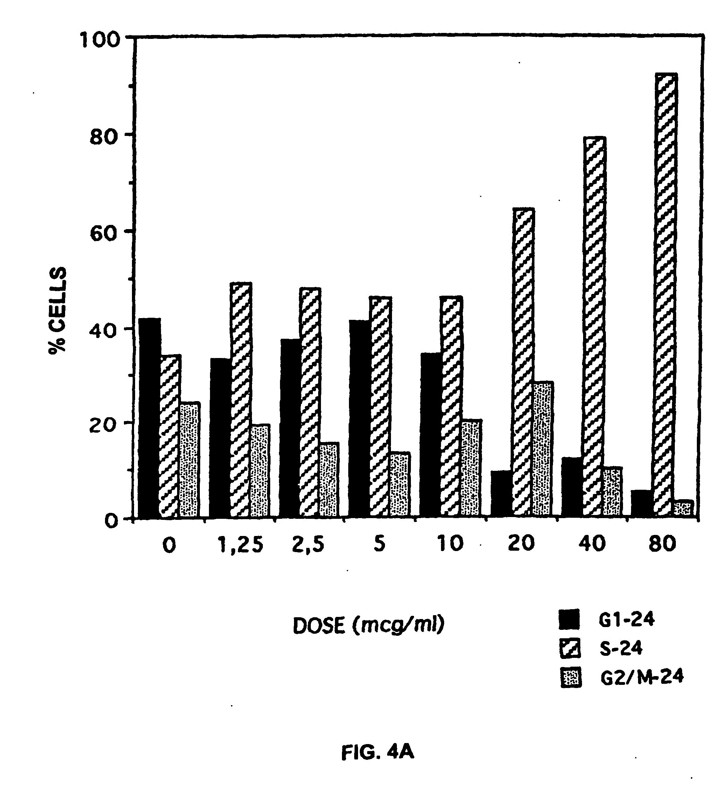

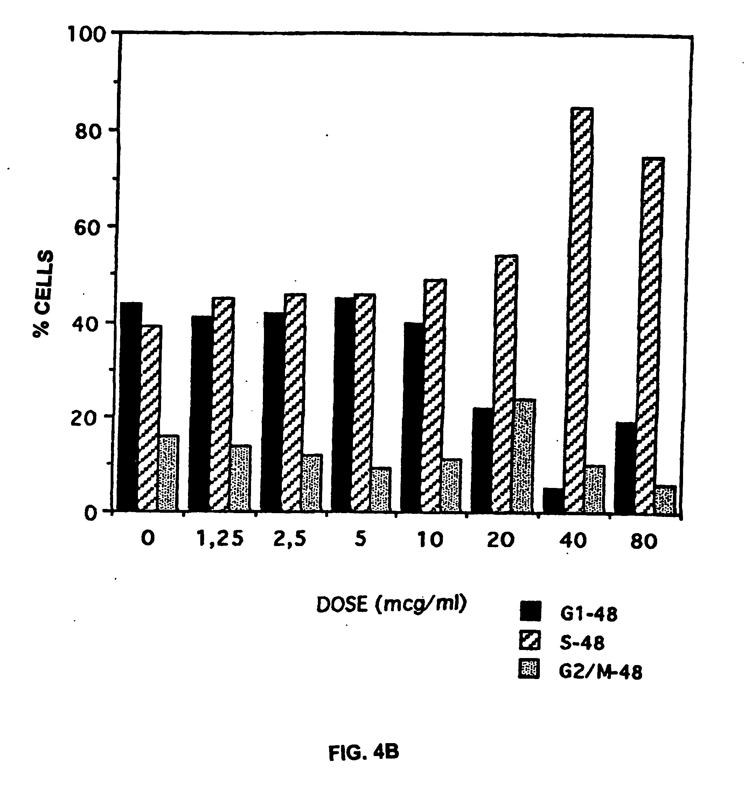

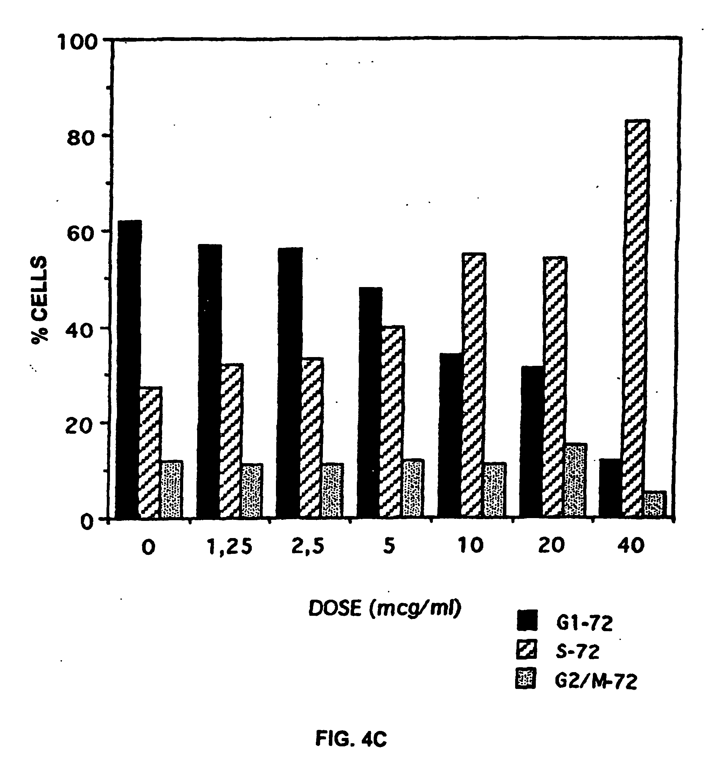

Eventually, C-Res has proved to be particularly active in inducing variations in the cellular cycle of M 14 cells (Fig. 4). Indeed, after only 24h of cell incubation with C-Res and starting from a dose of 1.25 mcg/ml, a significant block of the cellular cycle on S/G2M level can be observed, with the consequent increase of the percentage of cells in S stage. Said block remains, at higher doses, constant for the following hours.

These results clearly indicate that C-Res can perform an anti-tumoral activity towards cells belonging to the line of malignant melanoma M14. Said activity can be observed in said cells through i) the inhibition of growth depending on dose and time of incubation, ii) the induction of apoptosis depending on dose and time of incubation, iii) the induction of mortality depending on dose and time of incubation, iiii) the induction of variations in the cellular cycle depending on dose and time of incubation, in particular the induction of a block of the cellular cycle on S/G2M level.

### Example 12

### Anti-tumoral effects of cis-resveratrol on PAR-MEL cells (malignant melanoma resisting to chemotherapeutic treatments)

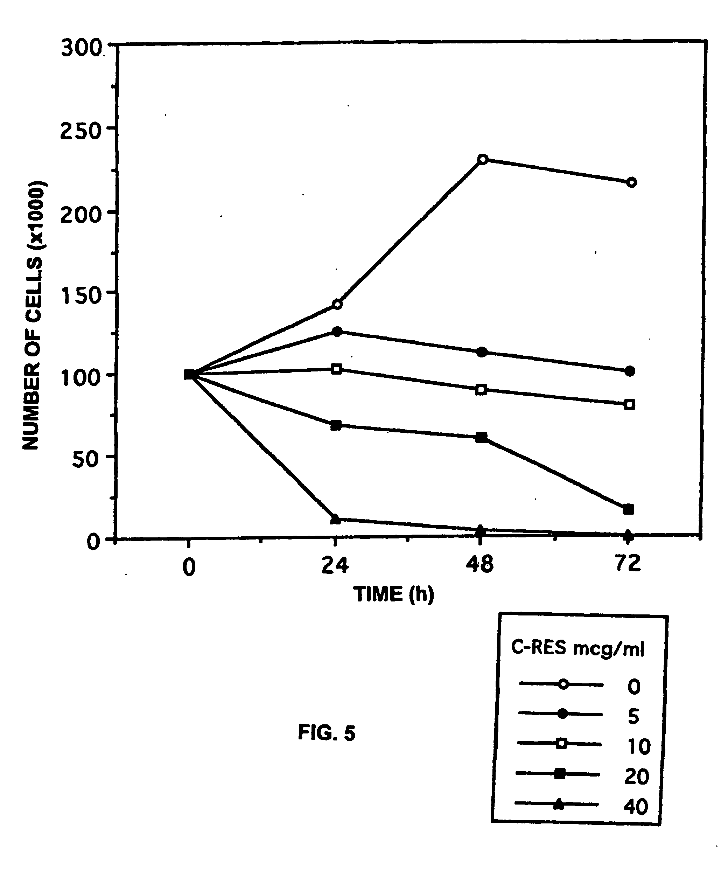

Cells belonging to the PAR-MEL tumor line (malignant melanoma resisting to chemotherapeutic treatments) have been incubated for various periods of time with 5, 10, 20 and 40 mcg/ml of C-Res. The results of the experiments show that C-Res can inhibit the growth of said cells. In particular, data show (Fig. 5) that in a dose range between 5 and 10 mcg/ml C-Res performs a significant cytostatic activity, whereas at a dose of 20 and 40 mcg/ml the compound has a cytotoxic activity.

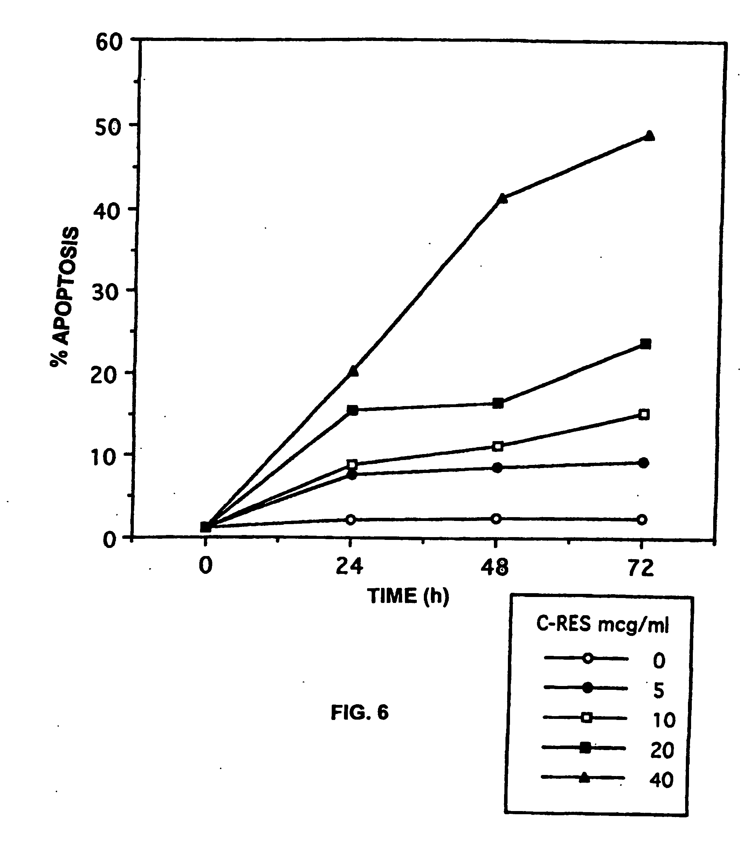

Similarly to what has been observed for M14 cells, the results of the cytofluorometric analysis of the DNA content of cells marked with PI show that C-Res induces an increase in the number of apoptotic cells depending on the dose and drug exposition time (Fig. 6). The results, anyway, show a higher sensitivity of said cells, with respect to M14 line, to the apoptotic effect of C-Res. Indeed, at a dose of 40 mcg/ml the substance has proved to be able to induce apoptosis in 50% of the cells after 72h of incubation.

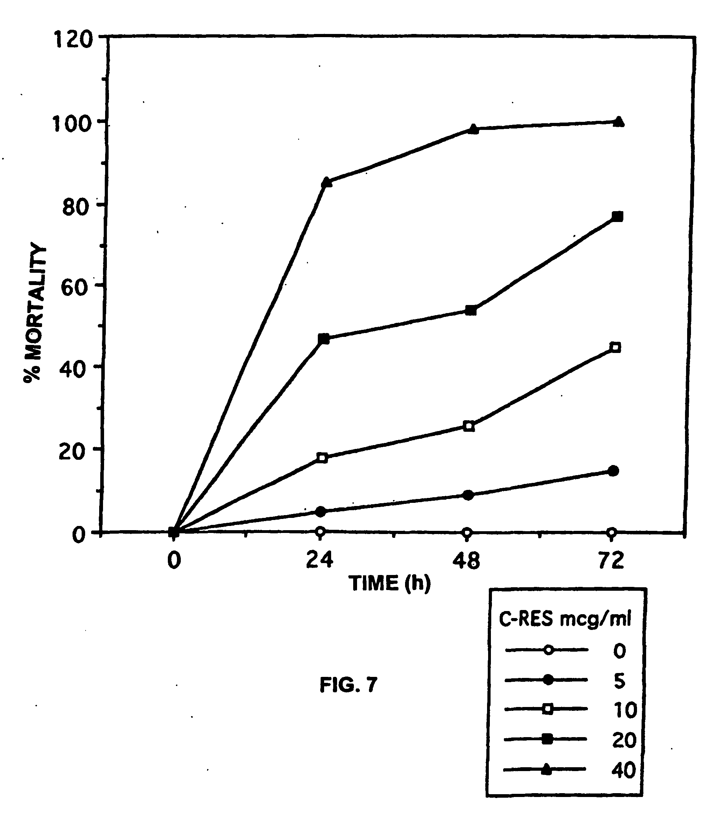

As far as the activity of C-Res on cell mortality (Fig. 7) is concerned, the results show that said activity is related to time and dose. Only for the dose of 40 mcg/ml the plateau is reached after only 24h of incubation.

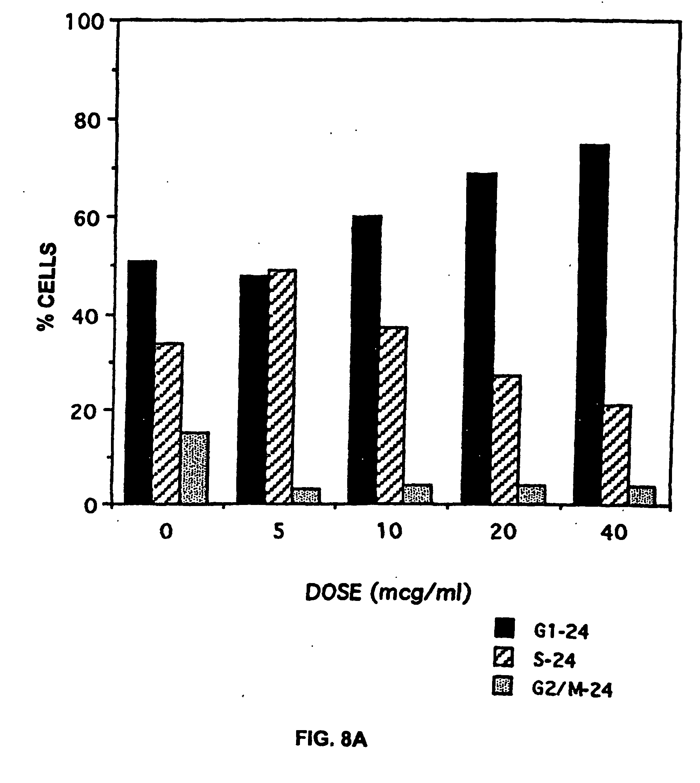

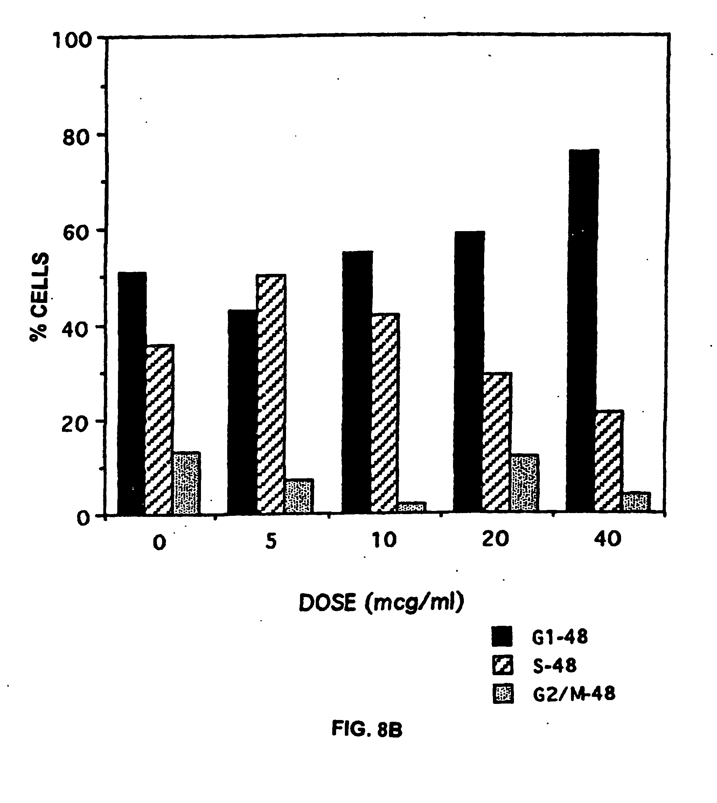

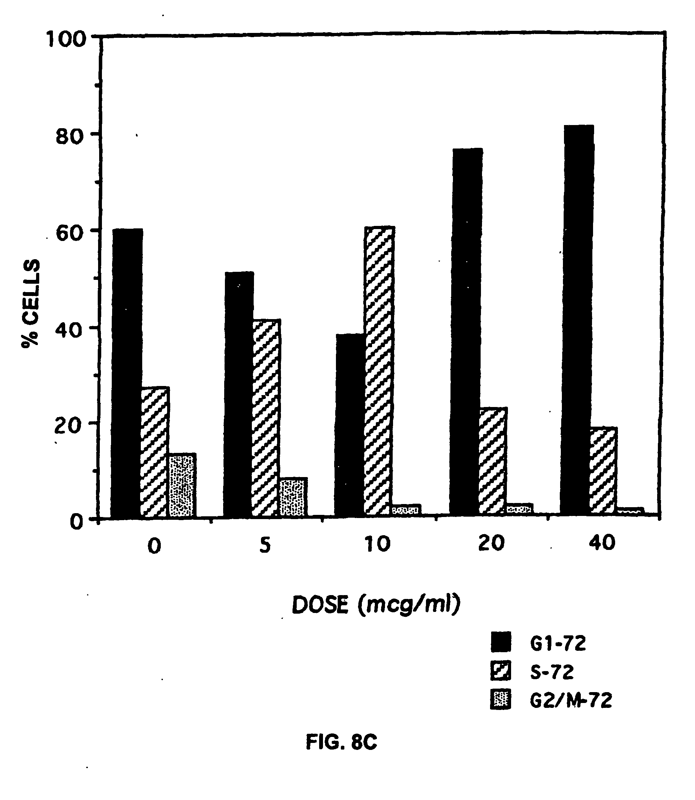

As far as the effects on the cellular cycle (Fig. 8) are concerned, after only 24h of incubation of PAR-MEL cells with C-Res and starting from a dose of 5 mcg/ml, a significant block of the cellular cycle on G1-S/G2M level can be observed, with a consequent increase in the percentage of cells in S stage and at higher doses in G1 stage. Said block remains constant for the following hours.

These results clearly indicate that C-Res can perform an anti-tumoral activity towards cells belonging to the line of malignant melanoma PAR-MEL resistent to chemotherapeutic treatments. Said activity can be observed in said cells through i) the inhibition of growth depending on dose and time of incubation, ii) the induction of apoptosis depending on dose and time of incubation, iii) the induction of mortality depending on dose and time of incubation, iiii) the induction of variations in the cellular cycle depending on dose and time of incubation, in particular the induction of a block of the cellular cycle on G1-S/G2M level.

### Example 13

### Effects of cis-resveratrol on HT-29 cells (colorectal adenocarcinoma)

Cells belonging to the HT-29 tumor line (colorectal adenocarcinoma) have been incubated for 24, 48 and 72 hours with 5, 10, 20 and 40 mcg/ml of C-Res. The growth curves related to the cell line observed until 72 hours show that at doses of 20 and 40 mcg/ml C-Res induces an inhibition, partial and complete respectively, of tumor growth, whereas at doses of 5 and 10 mcg/ml C-Res does not induce any significant effect.

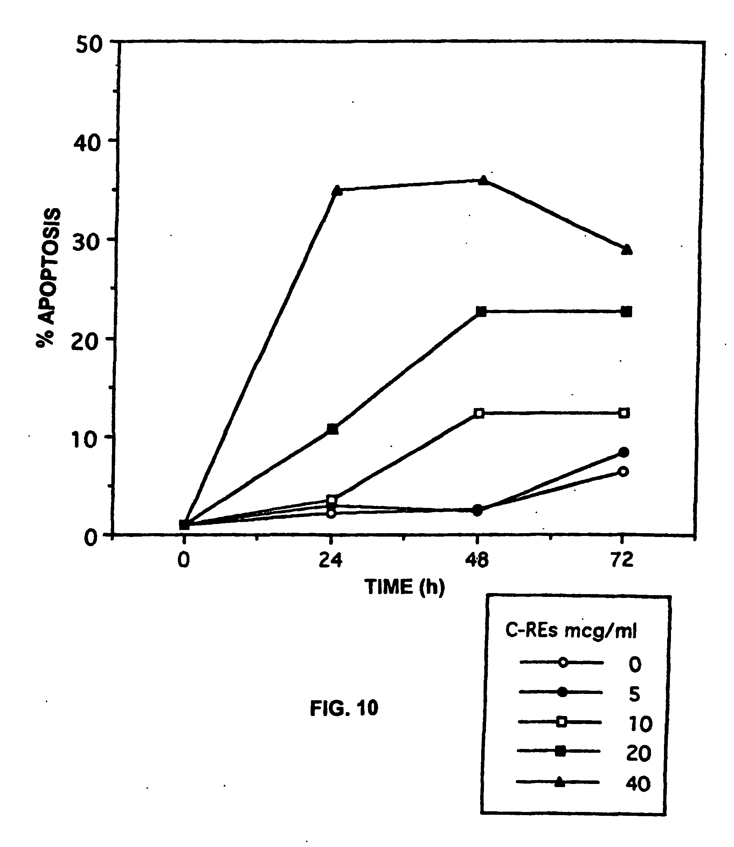

The results of the cytofluorometric analysis of the DNA content of cells marked with PI (Fig. 10) show an absence of measurable peaks of hypodiploidy, and therefore of apoptosis, at a dose of 5 mcg/ml for all times of incubation. For all other doses an increase of the effect related to the time of incubation can be observed.

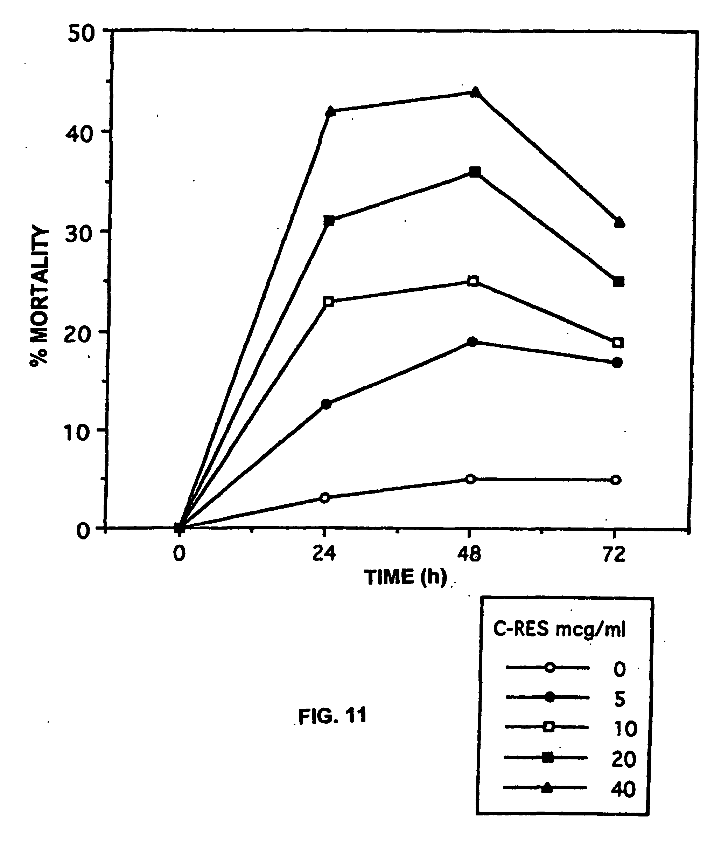

As far as the activity of C-Res on cell mortality (Fig. 11) is concerned, the results show that said activity is related to time and dose. For all doses the peak of activity is detected after 48 hours of incubation.

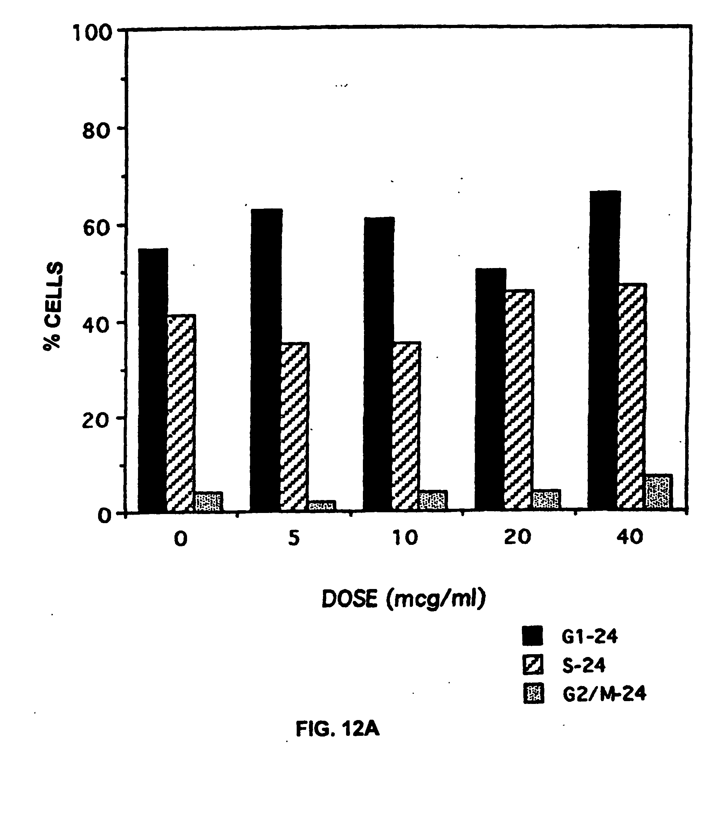

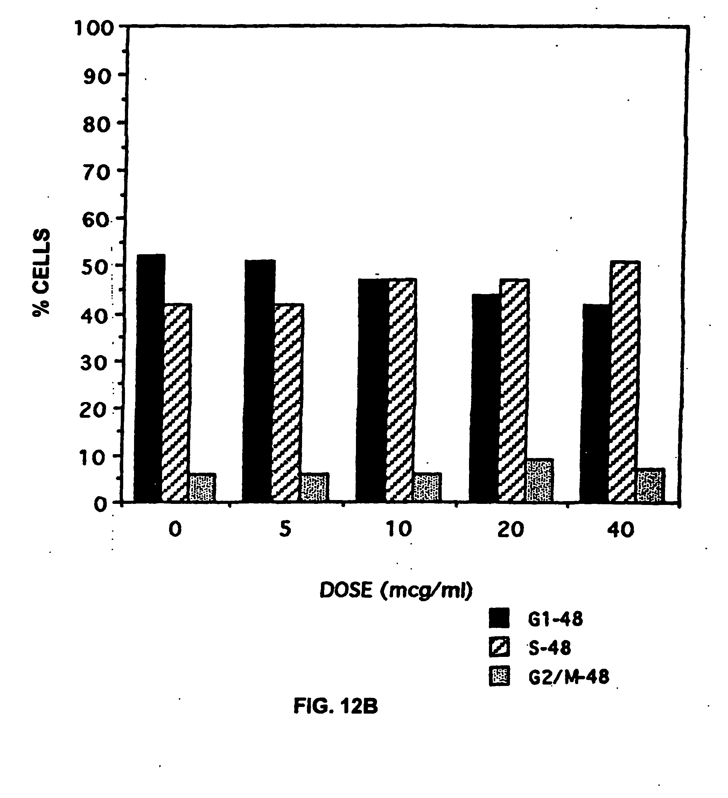

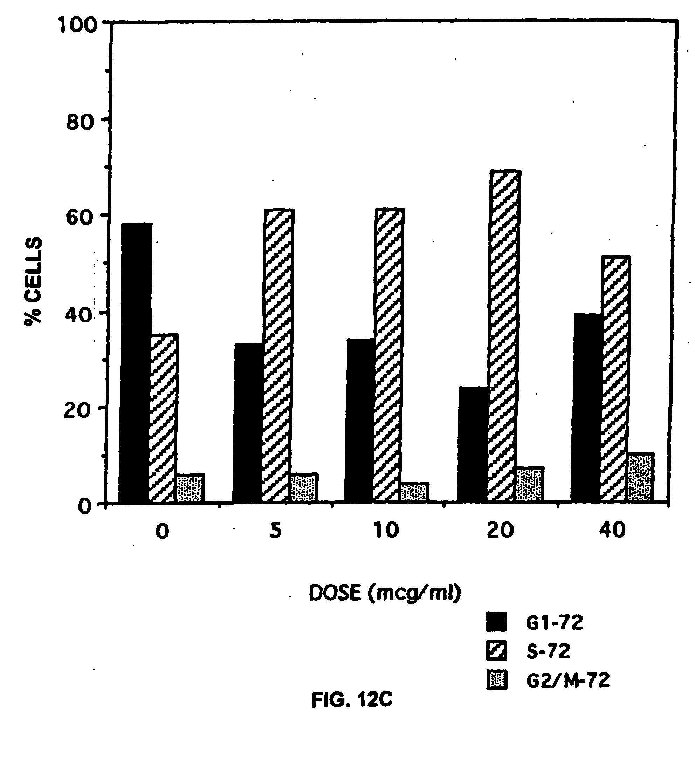

For these cells the effects on the cellular cycle (Fig. 12) are evident only after 48h of incubation starting from a dose of 10 mcg/ml and consist in a block of the cellular cycle on G1-S/G2M level.

These results clearly indicate that C-Res can perform an anti-tumoral activity towards cells belonging to the line of colorectal adenocarcinoma HT-29. Said activity can be observed in said cells through i) the inhibition of growth depending on dose and time of incubation, ii) the induction of apoptosis depending on dose and time of incubation, iii) the induction of mortality depending on dose and time of incubation, iiii) the induction of variations in the cellular cycle depending on dose and time of incubation, in particular the induction of a block of the cellular cycle on

G1-S/G2M level.

### Example 14

### Comparison between the anti-tumoral activity of cis-resveratrol (C-Res) ad trans-resveratrol (T-Res)

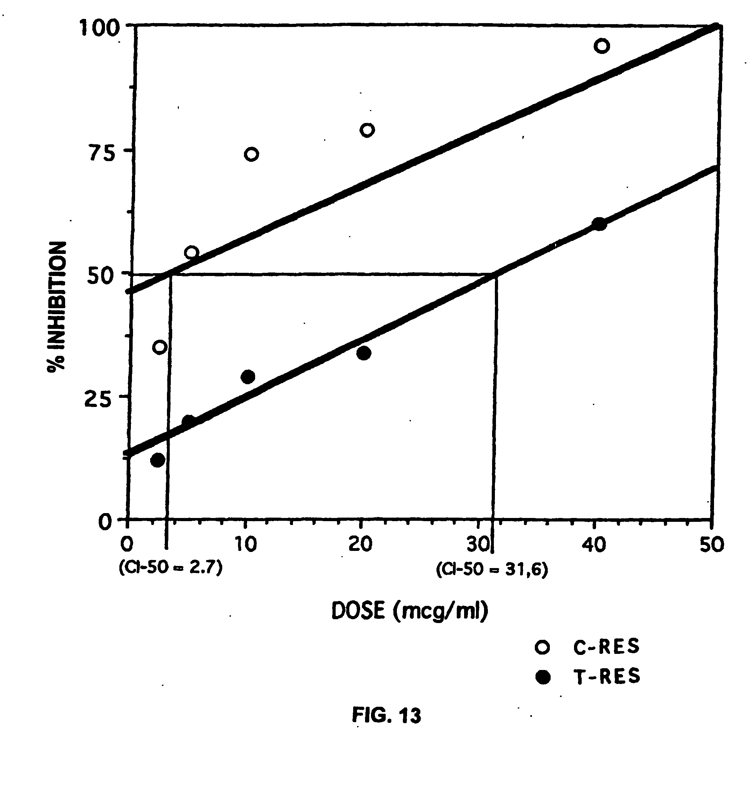

Cells belonging to M14 tumoral line (malignant melanoma) have been incubated for 72 h with 2.5, 5, 10, 20 and 40 mcg/ml of C-Res or T-Res. The results of the experiments have shown that the concentration inhibiting 50% of cell replication (Cl₅₀) corresponded to 2.7 and 31.6 mcg/ml for C-Res and T-Res respectively (Fig. 13). The analysis of the cellular cycle has confirmed that already at the lowest dose C-Res, but not T-Res, could induce cell accumulation in stage G1/S. These data indicate that the specific activity of C-Res is about 12 times higher that that of T-Res.

## Claims

1. Products selected among: H-gnetine, r-2-viniferine, r-viniferine, corresponding pharmaceutically acceptable salts, esters, amides, prodrugs and mixtures for use in the pharmaceutical field.

2. Pharmaceutical compositions comprising as active agent at least one of the compounds according to claim 1 together with suitable excipients.

3. Pharmaceutical compositions comprising as active agent at least one of the compounds according to claim 1 in form of tablets, suppositories, pills, capsules, powders, liquids, suspensions, creams, ointments or lotions.

4. Pharmaceutical compositions comprising as active agent at least one of the compounds according to claim 1 which can be administered by oral, parenteral (subcutaneous, intravenous, intramuscular injection), transdermic, rectal, nasal, buccal, topical way or by a controlled-release implant.

5. Pharmaceutical compositions comprising as active agent at least one of the compounds according to claim 1 with anti-tumoral activity, which can be administered at doses between 0.0001 and 20 mg/kg/die of the active agent.

6. Pharmaceutical compositions comprising as active agent at least one of the compounds according to claim 1 with cytostatic activity.

7. Pharmaceutical compositions comprising as active agent at least one of the compounds according to claim 1 with cytotoxic activity.

8. Use of at least one of the compounds selected in the group of: H-gnetine, r-2-viniferine, r-viniferine, corresponding pharmaceutically acceptable salts, esters, amides, prodrugs and mixtures thereof for the manufacture of a medicament for treating tumors.

9. Use of at least one of the compounds selected in the group of: Cis-Resveratrol, glucosidated Cis-Resveratrol, ε-viniferine, H-gnetine, r-2-viniferine, r-viniferine, hopeaphenol, Ampelopepsin A and glucosidated Trans-Resveratrol, corresponding pharmaceutically acceptable salts, esters, amides, prodrugs and mixtures thereof for the manufacture of a medicament for treating colorectal adenocarcinoma.

10. Use of at least one of the compounds selected in the group of: Cis-Resveratrol, glucosidated Cis-Resveratrol, ε-viniferine, H-gnetine, r-2-viniferine, r-viniferine, hopeaphenol, Ampelopepsin A and glucosidated Trans-Resveratrol, corresponding pharmaceutically acceptable salts, esters, amides, prodrugs and mixtures thereof for the manufacture of a medicament for treating melanoma.

11. Use according to claims 8 10 in which the medicament is in form of tablets, suppositories, pills, capsules, powders, liquids, suspensions, creams, ointments or lotions.

12. Use according to claims 8 10 in which the medicament is administered by oral, parenteral (subcutaneous, intravenous, intramuscular injection), transdermic, rectal, nasal, buccal, topical way or by a controlled-release implant.

13. Use according to claims 8 10 in which the medicament is administered at doses between 0.0001 and 20 mg/kg/die of the active agent.

## Patentansprüche

1. Produkte ausgewählt unter: H-Gnetin, r-2-Viniferin, r-Viniferin, entsprechenden pharmazeutisch akzeptablen Salzen, Estern, Amiden, Prodrugs und Mischungen für die Verwendung im pharmazeutischen Bereich.

2. Pharmazeutische Zusammensetzungen umfassend als Wirkstoff mindestens eine der Verbindungen gemäß Anspruch 1 zusammen mit geeigneten Trägerstoffen.

3. Pharmazeutische Zusammensetzungen umfassend als Wirkstoff mindestens eine der Verbindungen gemäß Anspruch 1 in Form von Tabletten, Suppositorien, Pillen, Kapseln, Pulvern, Flüssigkeiten, Suspensionen, Cremes, Salben oder Lotionen.

4. Pharmazeutische Zusammensetzung umfassend als Wirkstoff mindestens eine der Verbindungen gemäß Anspruch 1, welche über die orale, parenterale (subkutane, intravenöse, intramuskuläre Injektion), transdermale, rektale, nasale, bukkale oder topische Route verabreicht wird oder über ein Implantat zur kontrollierten Freisetzung.

5. Pharmazeutische Zusammensetzungen umfassend als Wirkstoff mindestens eine der Verbindungen gemäß Anspruch 1 mit Anti-Tumor-Wirkung, welche in aktiven Wirkstoffdosierungen von 0,0001 bis 20 mg/kg/die gegeben wird.

6. Pharmazeutische Zusammensetzungen umfassend als Wirkstoff mindestens eine der Verbindungen gemäß Anspruch 1 mit zytostatischer Aktivität.

7. Pharmazeutische Zusammensetzungen umfassend als Wirkstoff mindestens eine der Verbindungen gemäß Anspruch 1 mit zytotoxischer Aktivität.

8. Verwendung von mindestes einer der Verbindungen ausgewählt aus der Gruppe enthaltend: H-Gnetin, r-2-Viniferin, r-Viniferin, entsprechenden pharmazeutisch akzeptablen Salzen, Estern, Amiden, Prodrugs und Mischungen davon für die Herstellung eines Medikamentes zur Tumorbehandlung.

9. Verwendung von mindestes einer der Verbindungen ausgewählt aus der Gruppe enthaltend: Cis-Resveratrol, glukosidiertes Cis-Resveratrol, ε-Viniferin, H-Gnetin, r-2-Viniferin, r-Viniferin, Hopeaphenol, Ampelopepsin A und glukosidiertes Trans-Resveratrol, entsprechende pharmazeutisch akzeptable Salze, Ester, Amide, Prodrugs und Mischungen davon für die Herstellung eines Medikamentes zur Behandlung von kolorektalen Adenokarzinomen.

10. Verwendung von mindestes einer der Verbindungen ausgewählt aus der Gruppe enthaltend: Cis-Resveratrol, glukosidiertes Cis-Resveratrol, ε-Viniferin, H-Gnetin, r-2-Viniferin, r-Viniferin, Hopeaphenol. Ampelopepsin A und glukosidiertes Trans-Resveratrol, entsprechende pharmazeutisch akzeptable Salze, Ester, Amide, Prodrugs und Mischungen davon für die Herstellung eines Medikamentes zur Behandlung von Melanomen.

11. Verwendung gemäß den Ansprüchen 8-10, in welchen das Medikament in Form von Tabletten, Suppositorien, Pillen, Kapseln, Pulvem, Flüssigkeiten, Suspensionen, Cremes, Salben oder Lotionen vorliegt.

12. Verwendung gemäß den Ansprüchen 8-10, in welchen das Medikament über die orale, parenterale (subkutane, intravenöse, intramuskuläre Injektion), transdermale, rektale, nasale, bukkale oder topische Route verabreicht wird oder über ein Implantat zur kontrollierten Freisetzung.

13. Verwendung gemäß den Ansprüchen 8-10, in welchen das Medikament in aktiven Wirkstoffdosierungen von 0,0001 bis 20 mg/kg/die verabreicht wird.

## Revendications

1. Produits choisis parmi : la H-gnétine, la r-2-viniférine, la r-viniférine, des sels, des esters, des amides, des promédicaments pharmaceutiquement acceptables et des mélanges correspondants pour utilisation dans le domaine pharmaceutique.

2. Compositions pharmaceutiques comprenant en tant qu'agent actif au moins l'un des composés selon la revendication 1 conjointement avec des excipients adaptés.

3. Compositions pharmaceutiques comprenant en tant qu'agent actif au moins l'un des composés selon la revendication 1 sous forme de comprimés, de suppositoires, de pilules, de gélules, de poudres, de liquides, de suspensions, de crèmes, de pommades ou de lotions.

4. Compositions pharmaceutiques comprenant en tant qu'agent actif au moins l'un des composés selon la revendication 1 qui peuvent être administrées par voie orale, parentérale (injection sous-cutanée, intraveineuse, intramusculaire), transdermique, rectale, nasale, buccale, locale ou par un implant à libération contrôlée.

5. Compositions pharmaceutiques comprenant en tant qu'agent actif au moins l'un des composés selon la revendication 1 ayant une activité antitumorale, qui peuvent être administrées à des doses comprises entre 0,0001 et 20 mg/kg/die de l'agent actif.

6. Compositions pharmaceutiques comprenant en tant qu'agent actif au moins l'un des composés selon la revendication 1 ayant une activité cytostatique.

7. Compositions pharmaceutiques comprenant en tant qu'agent actif au moins l'un des composés selon la revendication 1 ayant une activité cytotoxique.

8. Utilisation d'au moins l'un des composés choisi dans le groupe consistant en : la H-gnétine, la r-2-viniférine, la r-viniférine, des sels, des esters, des amides, des promédicaments pharmaceutiquement acceptables et des mélanges correspondants pour la fabrication d'un médicament pour traiter des tumeurs.

9. Utilisation d'au moins l'un des composés choisi dans le groupe consistant en : le cis-resvératrol, le cis-resvératrol glucosidé, la ε-viniférine, la H-gnétine, la r-2-viniférine, la r-viniférine, l'hopéaphénol, l'ampélopepsine A et le trans-resvératrol glucosidé, des sels, des esters, des amides, des promédicaments pharmaceutiquement acceptables et des mélanges correspondants de ceux-ci pour la fabrication d'un médicament pour traiter l'adénocarcinome colorectal.

10. Utilisation d'au moins l'un des composés choisi dans le groupe consistant en : le cis-resvératrol, le cis-resvératrol glucosidé, la ε-viniférine, la H-gnétine, la r-2-viniférine, la r-viniférine, l'hopéaphénol, l'ampélopepsine A et le trans-resvératrol glucosidé, des sels, des esters, des amides, des promédicaments pharmaceutiquement acceptables et des mélanges correspondants de ceux-ci pour la fabrication d'un médicament pour traiter le mélanome.

11. Utilisation selon les revendications 8 à 10 dans laquelle le médicament est sous la forme de comprimés, de suppositoires, de pilules, de gélules, de poudres, de liquides, de suspensions, de crèmes, de pommades ou de lotions.

12. Utilisation selon les revendications 8 à 10 dans laquelle le médicament est administré par voie orale, parentérale (injection sous-cutanée, intraveineuse, intramusculaire), transdermique, rectale, nasale, buccale, locale ou par un implant à libération contrôlée.

13. utilisation selon les revendications 8 à 10 dans laquelle le médicament est administré à des doses comprises entre 0,0001 et 20 mg/kg/die de l'agent actif.
